(19) Europäisches Patentamt
European Patent Office
Office européen des brevets

(11) EP 3 236 920 B1

(12) # EUROPÄISCHE PATENTSCHRIFT

(45) Veröffentlichungstag und Bekanntmachung des Hinweises auf die Patenterteilung:
**04.10.2023 Patentblatt 2023/40**

(21) Anmeldenummer: **15820092.3**

(22) Anmeldetag: **17.12.2015**

(51) Internationale Patentklassifikation (IPC):
***A61K 8/34*** $^{(2006.01)}$ ***A61K 8/49*** $^{(2006.01)}$
***A61Q 19/00*** $^{(2006.01)}$ ***A61P 17/10*** $^{(2006.01)}$
***A61Q 19/10*** $^{(2006.01)}$ ***A61Q 17/00*** $^{(2006.01)}$
***A61K 45/06*** $^{(2006.01)}$ ***A61K 31/047*** $^{(2006.01)}$
***A61K 31/08*** $^{(2006.01)}$ ***A61K 31/444*** $^{(2006.01)}$

(52) Gemeinsame Patentklassifikation (CPC):
(C-Sets verfügbar)
**A61Q 19/10; A61K 8/345; A61K 8/4926; A61K 31/047; A61K 31/08; A61K 31/444; A61K 45/06; A61P 17/10; A61Q 17/005; A61Q 19/00;** A61K 2800/28 (Forts.)

(86) Internationale Anmeldenummer:
**PCT/EP2015/080127**

(87) Internationale Veröffentlichungsnummer:
**WO 2016/102288 (30.06.2016 Gazette 2016/26)**

(54) **ANTIMIKROBIELLE ZUBEREITUNG UMFASSEND BISPYRIDINIUMALKANE, 1- ODER 2-(C1 BIS C24-ALKYL)-GLYCERINETHER UND ALKAN-1,2-DIOLE**

ANTIMICROBIAL PREPARATION COMPRISING BISPYRIDINIUM ALKANES, 1- OR 2-(C1 TO C24 ALKYL) GLYCEROL ETHER AND ALKANE-1,2-DIOLS

PRÉPARATION ANTIMICROBIENNE COMPRENANT DES ALCANES DE BISPYRIDINIUM, DES ÉTHERS DE 1- OU 2-(C1 À C24-ALKYL)-GLYCÉROL ET DES ALCANE-1,2-DIOLS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.12.2014 DE 102014226763**
**22.12.2014 DE 102014226746**
**16.12.2015 DE 102015225398**

(43) Veröffentlichungstag der Anmeldung:
**01.11.2017 Patentblatt 2017/44**

(73) Patentinhaber: **Beiersdorf AG**
**20253 Hamburg (DE)**

(72) Erfinder:
- **WEETS, Gudrun**
  **22527 Hamburg (DE)**
- **GRÖNNIGER, Elke**
  **22303 Hamburg (DE)**
- **VON WEDEL-PARLOW, Magdalena**
  **20251 Hamburg (DE)**
- **TRAUPE, Bernd**
  **24568 Kaltenkirchen (DE)**

(74) Vertreter: **Beiersdorf AG**
**Patentabteilung**
**Unnastraße 48**
**20245 Hamburg (DE)**

(56) Entgegenhaltungen:
**WO-A1-2008/046791 WO-A1-2013/167220**
**DE-A1- 10 028 638 DE-A1- 10 341 179**
**DE-A1-102005 002 643 DE-A1-102008 011 692**
**DE-A1-102010 044 785 DE-A1-102010 044 787**
**DE-C1- 4 240 674 DE-T2- 60 129 705**
**US-A- 6 123 953**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

(Forts. nächste Seite)

• **FISHMAN H: "A WIDE ARRAY OF INGREDIENTS AT NYSCC SUPPLIERS' DAY", HAPPI HOUSEHOLD AND PERSONAL PRODUCTS INDUSTRY, RODMAN PUBLISHING, RAMSEY, NJ, US, Juli 2011 (2011-07), Seite 2pp, XP009188817, ISSN: 0090-8878**

Bemerkungen:

Die Akte enthält technische Angaben, die nach dem Eingang der Anmeldung eingereicht wurden und die nicht in dieser Patentschrift enthalten sind.

(52) Gemeinsame Patentklassifikation (CPC): (Forts.)

C-Sets
**A61K 31/047, A61K 2300/00;**
**A61K 31/08, A61K 2300/00;**
**A61K 31/444, A61K 2300/00**

## Beschreibung

Die Erfindung betrifft eine dermatologische und/oder kosmetische Zubereitung gemäß Anspruch 1, mit antimikrobieller Wirkung gegen *Propionibacterium acnes.*

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen, da ein sympathisches Erscheinungsbild ihr Selbstwertgefühl und die Anziehungskraft auf ihre Mitmenschen erhöht. Einen wesentlichen Anteil an einem schönen und attraktiven Äußeren haben dabei der Zustand und das Aussehen der Haut.

Bei fettig-öliger und unreiner Haut, als welche man den Übergangszustand zwischen der gesunden normalen und der krankhaft veränderten Aknehaut bezeichnet, produziert die Haut erhöhte Mengen an Talg (Seborrhoe). Dieser dient zahlreichen Mikroorganismen, insbesondere *Propionibacterium acnes* und *Pityrosporum*-Arten als idealer Nährboden. Die Mikroorganismen zersetzen den Talg zu Glycerin und Fettsäuren, wodurch die Talgdrüsen zu erhöhter Produktion angeregt und die Follikelwandungen in der Haut angegriffen und zerstört werden. Dies ruft Entzündungen in der Haut (Pickel, Pusteln, Knoten, Zysten) hervor, welche oft nur narbig ausheilen, wodurch das optische Erscheinungsbild des an unreiner Haut leidenden Menschen dauerhaft geschädigt wird (W. Umbach [Hrsg], Kosmetik, Entwicklung, Herstellung und Anwendung kosmetischer Mittel, 2.Aufl. Thieme Verlag, Stuttgart, 1995).

Unter Akne (im engeren Sinne *acne vulgaris*) versteht man verschiedene Erkrankungen der Talgdrüsenfollikel, die durch Sekretions- und Verhornungsstörungen nachfolgende Entzündungen und eventuelle Vernarbung gekennzeichnet sind. Die *acne vulgaris* tritt vor hauptsächlich in der Pubertät auf und konzentriert sich in der Regel auf talgdrüsenreiche Hautbezirke (Gesicht, Nacken, Brust, Rücken). Durch Talgdrüsenhyperplasie und eine Verhornungsstörung der Follikel kommt es zu deren Verstopfung mit Bildung von Komedonen und den für *acne vulgaris* typischen Effloreszenzen (Pschyrembel, Klinisches Wörterbuch, 258. Aufl., Walter de Gruyter-Verlag, Berlin, 1998).

Zur Prophylaxe und Behandlung von Akne werden effektive Wirkstoffe benötigt, die die Besiedlung der Haut mit Mikroorganismen wie *Propionibacterium acnes* reduzieren bzw. verhindern. Somit tragen die Wirkstoffe nicht nur zu einem verbesserten Hautzustand sondern auch zu einem verbesserten Wohlbefinden des Anwenders bei.

Typische eingesetzte Wirkstoffe mit antimikrobieller Wirkung sind beispielsweise starke Oxidationsmittel, wie z.B. Benzoylperoxid; alpha-Hydroxysäuren, wie z.B. Milchsäure oder Salicylsäure; sowie aliphatische Dicarbonsäuren, wie z.B. Azelainsäure. Problematisch ist allerdings, dass die antimikrobielle Wirkung dieser Wirkstoffe gegenüber *Propionibacterium acnes* nur moderat ist. Somit müssen verhältnismäßig hohe Konzentrationen - Benzoylperoxid z.B. mit bis zu 5 Gew.-% und Azelainsäure mit bis zu 20 Gew.-% - in kosmetischen und dermatologischen Formulierungen eingesetzt werden. Bedingt durch diese hohen Konzentrationen wird nachteilig die Haut sehr stark strapaziert, was sich insbesondere in einer starken Austrocknung der Haut äußert. Ferner sind starke Hautirritationen möglich, die auf eine Reduktion des pH-Werts der Haut bei Anwendung der beschriebenen Wirkstoffe zurückzuführen ist.

Alternativ ist die topische Anwendung von effektiv gegen *Propionibacterium acnes* wirkenden Antibiotika, wie Clindamycin, Erythromycin und Tetracyclin, möglich. Insbesondere aufgrund des in der klinischen Praxis immer häufiger zu beobachtenden Auftretens resistenter *Propionibacterium acnes* Stämme sowie deren unspezifischem Wirkungsspektrum, welches zu einer starken Beeinträchtigung der gesunden humanen Mikroflora führt, geraten auch die in der Akne-Therapie geläufigen Antibiotika zunehmend ins Licht der Kritik.

Wünschenswert sind daher effektive antibiotikafreie Wirkstoffe oder Wirkstoffkombinationen, die das Wachstum und die Verbreitung von Akne hervorrufenden Bakterien auf der Haut, insbesondere von *Propionibakterium acnes* unterdrückt und gleichzeitig weniger oder nicht Haut irritierend wirken.

DE 102005002644 A1 offenbart eine Zusammensetzung umfassend Glycerinmonoalkylether, Bispyridiniumalkane und Polyole, welche eine effektive antimikrobielle Wirksamkeit gegen die Bakterien *Staphylococcus aureus* und *Pseudomonas aeruginosa* aufweisen. Bevorzugt wird als Glycerinmonoalkylether Ethylhexylglycerin, als Bispyridiniumalkan Octenidindihydrochlorid und als Polyol Glycerin eingesetzt.

Da sowohl *Staphylococcus aureus* als auch *Propionibakterium acnes* grampositive Bakterien sind, ist es naheliegend, dass eine Zusammensetzung umfassend Glycerinmonoalkylether, Bispyridiniumalkane und Polyole auch eine Wirksamkeit gegen *Propionibakterium acnes* aufweist. Dennoch sind Wirkstoffkombinationen einer verbesserten antimikrobiellen Wirksamkeit gegen *Propionibakterium acnes* wünschenswert.

Die Aufgabe der vorliegenden Erfindung bestand daher darin ausgehend von der in DE 102005002644 A1 offenbarten Zusammensetzung eine Wirkstoffkombination bereitzustellen, die eine verbesserte Wirksamkeit gegen *Propionibakterium acnes* aufweist. Des Weiteren bestand die Aufgabe darin eine Wirkstoffkombination bereitzustellen, die weniger oder nicht Haut irritierend wirkt und die Besiedlung der Haut mit Mikroorganismen, wie *Propionibacterium acnes,* reduziert.

Überraschend für den Fachmann wurde nun gefunden, dass eine Wirkstoffkombination umfassend ein oder mehrere Bispyridiniumalkane, ein oder mehrere 1- oder 2-($C_1$ bis $C_{24}$-Alkyl)-glycerinether (Glycerinmonoalkylether), ein oder mehrere Polyole mit einer Kettenlänge von maximal 4 Kohlenstoffatomen und ein oder mehreren Alkan-1,2-diolen

mit einer Kettenlänge von 8 bis 12 C-Atomen, dadurch gekennzeichnet, dass das Gewichtsverhältnis der Bispyridiniumalkane zu den 1- oder 2-($C_1$ bis $C_{24}$-Alkyl)-glycerinethern von 3:1 bis 1,7:1 beträgt, eine effektive antimikrobielle Wirkung gegen *Propionibacterium acnes* aufweist. Überraschend hat sich gezeigt, dass durch den zusätzlichen Einsatz eines oder mehrerer Alkan-1,2-diole mit einer Kettenlänge von 8 bis 12 C-Atomen eine synergistisch verbesserte antimikrobielle Wirkung gegen *Propionibacterium acnes* auftritt.

Die antibakterielle Wirkung von Alkan-1,2-diolen an sich ist weitreichend bekannt.

Beispielsweise beschreibt JP 20022003330 die antibakterielle Wirkung einer Kombination von Alkan-1,2-diolen und Ascorbinsäureestern.

Die WO 03000220 A2 beschreibt die Verwendung von Decan-1,2-diol gegen Körpergeruch verursachende Bakterien. Nicht zuletzt beschreibt die US 6123953 A die Verwendung von Alkan-1,2-diolen zur Inaktivierung von Mikroorganismen auf der Haut durch den Einsatz von Octan-1,2-diol. In dieser Schrift wird auch die Wirkung von Octan-1,2-diol gegen Akne erwähnt.

EP 1915982 A1 beschreibt die Verwendung von 1,2-Decandiol zur Reduktion der Sebumkonzentration der Haut und als Mittel zur Behandlung von Akne. Ferner wird in EP 1598064 A1 eine Wirkstoffkombination aus alpha- und/oder beta-Hydroxysäuren und Alkan-1,2-Diolen zur Behandlung von Akne vorgeschlagen, wobei beschrieben wird, dass die Kombination von 1% Milchsäure mit 0,2% Decan-1,2-diol eine verbesserte antibakterielle Wirksamkeit der Kombination in Bezug auf *Propionibakterium acnes* gegenüber der Einzelbestandteile, insbesondere von 0,2% Decan-1,2-diol aufweist. Bei der alleiniger Gabe von 0,2% Decan-1,2-diol ist so gut wie keine antibakterielle Wirkung im Suspensionstest ersichtlich. Da sich Milchsäure allerdings chemisch grundlegend von Glycerinmonoalkylether, Bispyridiniumalkane und Polyole unterscheidet konnte auch diese Offenbarung keinen Hinweis auf die vorteilhaften Eigenschaften der Erfindung hinweisen.

Ferner offenbart das Dokument WO 2013/167220 A1 kosmetische Zubereitungen welche als Anti-Akne Gesichtsreinigungsgele eingesetzt werden können. Im speziellen wird in Beispiel 8 eine Zubereitung offenbart, welche Octenidin, Ethylhexylglycerin, 1,2 Decandiol sowie Polyole mit einer Kettenlänge von maximal 4 Kohlenstoffatomen offenbart. Jedoch unterscheidet sich die offenbarte Zubereitung von der vorliegenden Erfindung in den eingesetzten Gewichtsverhältnissen der Bispyridiniumalkane und der 1- oder 2-(C1 bis C24-Alkyl)-glycerinether.

Des Weiteren kennt der Fachmann das Dokument DE 102008011692 A1, welches Zusammensetzungen aus Bispyridiniumalkanen, Polyolen, und 1- oder 2-(C1 bis C24-Alkyl)-glycerinether offenbart. Es wird jedoch kein Hinweis auf die Gewichtsverhältnisse der Bispyridiniumalkane zu den 1- oder 2-(C1 bis C24-Alkyl)-glycerinether gemäß der vorliegenden Erfindung gegeben.

Gegenstand der Erfindung ist daher eine dermatologische und/oder kosmetische Zubereitung, umfassend

a) ein oder mehrere Bispyridiniumalkane,
b) ein oder mehrere 1- oder 2-($C_1$ bis $C_{24}$-Alkyl)-glycerinether (Glycerinmonoalkylether),
c) ein oder mehrere Polyole mit einer Kettenlänge von maximal 4 Kohlenstoffatomen, und
d) ein oder mehrere Alkan-1 ,2-diolen mit einer Kettenlänge von 8 bis 12 Kohlenstoffatomen,

dadurch gekennzeichnet ist, dass die dermatologische und/oder kosmetische Zubereitung frei von aluminiumhaltigen antitranspirant wirkenden Substanzen ist und dass das Gewichtsverhältnis der Bispyridiniumalkane zu den 1- oder 2-($C_1$ bis $C_{24}$-Alkyl)-glycerinethern von 3:1 bis 1,7:1 beträgt.

Frei von bedeutet dabei, dass der Anteil an diesen Stoffen, auf die verzichtet werden kann, weniger als 0,1 Gew.-%, bezogen auf die Gesamtmasse der Zubereitung, beträgt. Dadurch wird gewährleistet, dass Einschleppungen oder Verunreinigungen mit diesen Stoffen nicht als erfindungsgemäß frei von mitumfasst.

Die erfindungsgemäße dermatologische und/oder kosmetische Zubereitung kann gegen *Propionibakterium acnes* verwendet werden.

Wird in dieser Offenbarung der Ausdruck "gegen *Propionibakterium acnes"* verwendet, so bedeutet das, dass *Propionibacterium acnes* abgetötet wird und/oder das Wachstum und/oder die Ausbreitung des Bakteriums vermindert und/oder verhindert wird.

Ferner sei die nicht-therapeutische Verwendung der erfindungsgemäßen dermatologischen und/oder kosmetischen Zubereitung als Mittel zur kosmetischen Prophylaxe und/oder kosmetischen Behandlung von Akne erwähnt.

Die erfindungsgemäße dermatologische und/oder kosmetische Zubereitung kann zur Herstellung eines Medikaments gegen *Propionibakterium acnes* und/oder zur kosmetischen und/oder therapeutischen Behandlung von Akne verwendet werden.

Ferner ist die erfindungsgemäße dermatologische und/oder kosmetische Zubereitung bevorzugt frei von Triclosan.

Die erfindungsgemäße dermatologische und/oder kosmetische Zubereitung ist erfindungsgemäß frei von aluminiumhaltigen, antitranspirant wirksamen Substanzen.

Alle nachstehenden Gewichtsprozentangaben (Gew.-%) beziehen sich, sofern nicht anders angegeben auf

das Gesamtgewicht der erfindungsgemäßen dermatologischen und/oder kosmetischen Zubereitung.

Die erfindungsgemäße dermatologische und/oder kosmetische Zubereitung enthält ein oder mehrere Bispyridiniumalkane. Erfindungsgemäße Bispyridiniumalkane sind alle Substanzen der folgenden Formeln beziehungsweise Strukturen:

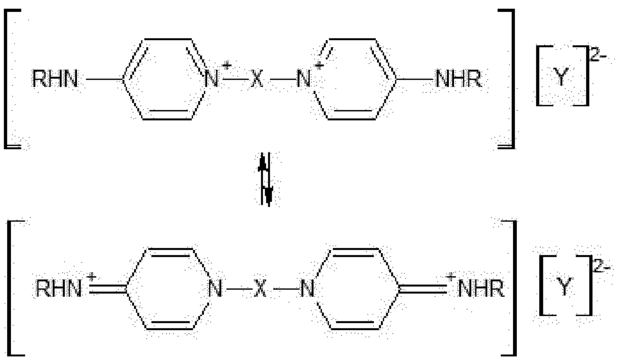
(I)
(II)

In Formel I und II steht X für eine Alkylkette mit einer Kettenlänge von 4 bis 18 Kohlenstoffatomen, R steht ebenfalls für Alkylgruppe mit 6 bis 18 Kohlenstoffatomen und Y steht für ein oder mehrere Anionen. Erfindungsgemäß besonders bevorzugt ist der Einsatz von Octenidindihydrochlorid (*N*-Octyl-1-[10-(4-octyliminopyridin-1-yl)decyl]pyridin-4-imin, *N,N'*-(Decan-1,10-diyldi-1(4*H*)-pyridyl-4-yliden)bis(octylammonium)dichlorid)) welches die folgenden Strukturen aufweisen kann:

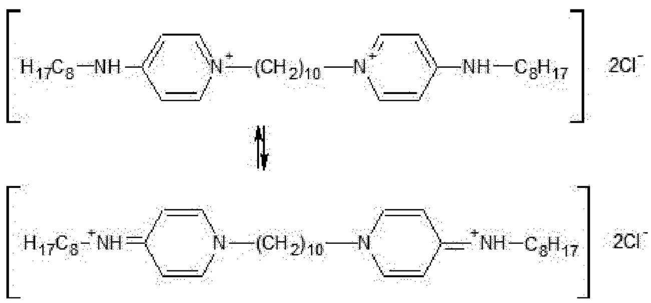

Die INCI-Bezeichnung für Octenidindihydrochlorid lautet Octenidine HCL.

Es ist insbesondere vorteilhaft im Sinne der vorliegenden Erfindung, wenn neben Octenidindihydrochlorid keine weiteren Bispyridiniumalkane in der erfindungsgemäßen dermatologischen und/oder kosmetischen Zubereitung enthalten sind.

Erfindungsgemäß sind Bispyridiniumalkane in einem Gesamtanteil von 0,001 Gew.-% bis 1,0 Gew.-%, bevorzugt von 0,01 Gew.-% bis 0,5 Gew.-%, insbesondere bevorzugt 0,05 Gew.-% bis 0,2 Gew.-% in der erfindungsgemäßen dermatologischen und/oder kosmetischen Zubereitung enthalten.

Die erfindungsgemäße dermatologische und/oder kosmetische Zubereitung enthält erfindungsgemäß ein oder mehrere 1- oder 2-($C_1$ bis $C_{24}$-Alkyl)-glycerinether. Unter diesen Verbindungen werden bevorzugt Propylglycerinether, Octylglycerinether, Decylglycerinether, Dodecylglycerinether, Octadecylglycerinether und/oder Hexadecylglycerinether gewählt.

Insbesondere bevorzugt ist als 1- oder 2-($C_1$ bis $C_{24}$-Alkyl)-glycerinether 1-(2-Ethylhexyl)glycerinether, nachfolgend als Ethylhexylglycerin (INCI Name) bezeichnet, zu wählen. Ethylhexylglycerin ist unter anderem unter dem Handelsnamen sensivia® SC 50 von der Fa. Schülke & Mayr GmbH zu beziehen.

Es ist insbesondere vorteilhaft im Sinne der vorliegenden Erfindung, wenn neben Ethylhexylglycerin keine weiteren 1- oder 2-($C_1$ bis $C_{24}$-Alkyl)-glycerinether in der erfindungsgemäßen dermatologischen und/oder kosmetischen Zubereitung enthalten sind.

Der Gesamtanteil der 1- oder 2-($C_1$ bis $C_{24}$-Alkyl)-glycerinether in der erfindungsgemäßen dermatologischen und/oder kosmetischen Zubereitung beträgt 0,001 Gew.-% bis 1,0 Gew.-%, bevorzugt 0,01 Gew.-% bis 0,5 Gew.-%, insbesondere bevorzugt 0,02 Gew.-% bis 0,1 Gew.-%.

Eine vorteilhafte Ausführungsform der Erfindung ist dadurch gekennzeichnet, dass das Gewichtsverhältnis der Bispyridiniumalkane zu den 1- oder 2-($C_1$ bis $C_{24}$-Alkyl)-glycerinethern im Bereich von 2,5:1 bis 1,7:1 beträgt.

Die erfindungsgemäße dermatologische und/oder kosmetische Zubereitung enthält erfindungsgemäß ein oder

mehrere Polyole mit einer Kettenlänge von maximal 4 Kohlenstoffatomen. Bevorzugt zu wählende Polyole mit einer Kettenlänge von maximal 4 Kohlenstoffatomen sind Glycerin (INCI-Bezeichnung auch Glycerol) und/oder Propan-1,2-diol (INCI-Bezeichnung: Propylene Glycol). Insbesondere bevorzugt ist Propan-1,2-diol als Polyole mit einer Kettenlänge von maximal 4 Kohlenstoffatomen zu wählen.

Polyole mit einer Kettenlänge von maximal 4 Kohlenstoffatomen werden in der erfindungsgemäßen dermatologischen und/oder kosmetischen Zubereitung als Lösungsmittel eingesetzt. An sich weisen die Polyole mit einer Kettenlänge von maximal 4 Kohlenstoffatomen kein oder nur ein geringe antimikrobielle Wirksamkeit gegen *Propionibakterium acnes* auf.

Es ist erfindungsgemäß insbesondere vorteilhaft, wenn die ein oder mehreren Bispyridiniumalkane, die ein oder mehreren 1- oder 2-($C_1$ bis $C_{24}$-Alkyl)-glycerinether und die ein oder mehreren Polyole mit einer Kettenlänge von maximal 4 Kohlenstoffatomen zusammen in einer separaten Lösung vorliegen und diese separate Lösung zur Herstellung der erfindungsgemäßen dermatologischen und/oder kosmetischen Zubereitung verwendet wird.

Geeignet ist daher ein Verfahren zur Herstellung der erfindungsgemäßen dermatologischen und/oder kosmetischen Zubereitung, wobei die ein oder mehreren Bispyridiniumalkane, die ein oder mehreren 1- oder 2-($C_1$ bis $C_{24}$-Alkyl)-glycerinether und die ein oder mehreren Polyole mit einer Kettenlänge von maximal 4 Kohlenstoffatomen zusammen in Form einer separaten Lösung der Zubereitung zugegeben werden. Dabei können die ein oder mehreren Alkan-1,2-diole mit einer Kettenlänge von 8 bis 12 Kohlenstoffatomen entweder vor, gleichzeitig oder nach dem Zugeben der separaten Lösung, umfassend Octenidindihydrochlorid, Ethylhexylglycerin und ein oder mehrere Polyole mit einer Kettenlänge von maximal 4 Kohlenstoffatomen, der dermatologischen und/oder kosmetischen Zubereitung zugegeben werden.

Die separate Lösung umfassend Octenidindihydrochlorid, Ethylhexylglycerin und ein oder mehrere Polyole mit einer Kettenlänge von maximal 4 Kohlenstoffatomen wird vorteilhaft der wässrigen Phase der dermatologischen und/oder kosmetischen Zubereitung zugegeben.

Bevorzugt umfasst eine solche separate Lösung Octenidindihydrochlorid in einem Anteil von 18 Gew.-% bis 22 Gew.-%, Ethylhexylglycerin in einem Anteil von 9 Gew.-% bis 11 Gew.-% und Polyole mit einer Kettenlänge von maximal 4 Kohlenstoffatomen, bevorzugt Propan-1,2-diol, in einem Anteil von 67 Gew.-% bis 73 Gew.-%, wobei die Gew.-% Angaben auf das Gesamtgewicht der Lösung bezogen sind. Eine derartige Lösung ist unter anderem unter dem Handelsnamen TPI 2007 von der Schülke & Mayr GmbH zu beziehen. TPI 2007 umfasst 20 Gew.-% Octenidindihydrochlorid, 10 Gew.-% Ethylhexylglycerin und 70 Gew.-% Propan-1,2-diol, bezogen auf das Gesamtgewicht der Lösung.

Erfindungsgemäß setzt sich die erfindungsgemäße dermatologische und/oder kosmetische Zubereitung aus ein oder mehreren Bispyridiniumalkanen, ein oder mehreren 1-oder 2-($C_1$ bis $C_{24}$-Alkyl)-glycerinethern, ein oder mehreren Polyole mit einer Kettenlänge von maximal 4 Kohlenstoffatomen und ein oder mehreren Alkan-1,2-diolen mit einer Kettenlänge von 8 bis 12 Kohlenstoffatomen zusammen. Als ein oder mehrere Alkan-1,2-diolen mit einer Kettenlänge von 8 bis 12 Kohlenstoffatomen werden bevorzugt Octan-1,2-diol, Decan-1,2-diol und/oder Dodecan-1,2-diol gewählt. Insbesondere bevorzugt ist der Einsatz von Decan-1,2-diol in der erfindungsgemäßen dermatologischen und/oder kosmetischen Zubereitung.

Es ist insbesondere vorteilhaft im Sinne der vorliegenden Erfindung, wenn neben Decan-1,2-diol keine weiteren Alkan-1,2-diolen mit einer Kettenlänge von 8 bis 12 Kohlenstoffatomen in der erfindungsgemäßen dermatologischen und/oder kosmetischen Zubereitung enthalten sind.

Alkan-1,2-diole mit einer Kettenlänge von 8 bis 12 Kohlenstoffatomen sind in einem Gesamtanteil von 0,01 Gew.-% bis 1 Gew.-%, bevorzugt von 0,05 Gew.-% bis 0,7 Gew.-% und insbesondere besonders von 0,1 Gew.-% bis 0,5 Gew.-% in der erfindungsgemäßen dermatologischen und/oder kosmetischen Zubereitung enthalten.

Die antimikrobielle Wirksamkeit der erfindungsgemäßen dermatologischen und/oder kosmetischen Zubereitung gegen *Propionibakterium acnes (P. acnes)* wurde zum Vergleich mit verschiedenen Formulierungen untersucht.

Dazu wurde das Bakterium *P. acnes (DSM* 1897) aus einem nach EN 12353 angelegten Cryoröhrchen auf einer entsprechenden Agarplatte (Cost-Agar) ausgestrichen und bei 37°C 5 Tage unter anaeroben Bedingungen bebrütet.

Eine Impföse der Bakterien wurde zu 10mL anaerobem Medium gegeben und im Impedanzgerät mit dem Programm PR37NT unter anaeroben Bedingungen inkubiert. Anschließend wurde diese Bakteriensuspension auf eine OD (optische Dichte) von 0,3-0,5 eingestellt. Die eingestellte Bakteriensuspension wurde dann 1:10 mit anaerobem Medium verdünnt, um eine Keimzahl von ungefähr $5*10^6$ zu gewährleisten. Diese Bakteriensuspension wurde anschließend für den Test eingesetzt.

Der Suspensionstest wurde per Hand durchgeführt. Hierzu wurden 500μL der zu testenden Wirkstofflösungen bzw. im Falle der Kontrollen 500 μl anaerobem Medium in Mikroreaktionsgefäßen vorgelegt. Anschließend wurden 500 μl der Bakteriensuspension hinzugegeben und gemischt. Zum Zeitpunkt $t_0$ wurde eine Probe aus den Kontrollen entnommen. Die Mikroreaktionsgefäße wurden nun im Thermoschüttler bei 37 °C inkubiert. Nach 24h bei 37°C wurden aus den Mikroreaktionsgefäßen im Thermoschüttler jeweils 100μL entnommen und zu 900μL Whitley Anaeroben Me-

dium pipettiert ($10^{-1}$-Verdünnung). Diese Verdünnungen wurden mittels Spiralplatter auf Agarplatten ausplattiert. Eine Ausnahme stellt hier die Kontrolle dar, die nochmal 1:100 verdünnt wurde ($10^{-3}$-Verdünnug). Bei den Kontrollen wurden beide Verdünnungen ausplattiert, einschließlich des Zeitpunkts T=0 Minuten. Zum Schluss wurden die Platten bei 37°C 5 Tage unter anaeroben Bedingungen bebrütet und die Zellzahl bestimmt.

In der Auswertung wurde der Reduktionsfaktor bestimmt. Der Reduktionsfaktor ist der Faktor, um den die Zellzahl (CFU) der Testsubstanz im Vergleich zur Kontrolle reduziert wird. Diese berechnen sich wie folgt:

$$Reduktionsfaktor = \frac{cfu(Kontrolle)}{cfu(Testsubstanz)}$$

Die zu testenden Wirkstofflösungen wurden hergestellt in dem die jeweiligen Testsubstanzen in einem Phosphatpuffer gelöst wurden. Der Phosphatpuffer enthielt 2,284 g/L Kaliumdihydrogenphosphat ($KH_2PO_4$) und 8,889 g/L Dinatriumhydrogenphosphat Dihydrat ($Na_2HPO_4 \cdot 2\ H_2O$). Der Rest restliche Anteil war Wasser.

Die Wirkstoffkonzentration der untersuchten Proben (A,B,C) betrug:

A: 0,2 μg/ml TPI 2007 (20 Gew.-% Octenidindihydrochlorid, 10 Gew.-% Ethylhexylglycerin und 70 Gew.-% Propan-1,2-diol)
B: 250 μg/ml Decan-1,2-diol
C: 250 μg/ml Decan-1,2-diol, 0,2 μg/ml TPI 2007 (20 Gew.-% Octenidindihydrochlorid, 10 Gew.-% Ethylhexylglycerin und 70 Gew.-% Propan-1,2-diol)

Umso größer der bestimmte Reduktionsfaktor wird, desto mehr nimmt die Keimzahl im Vergleich zur Kontrolle ab und desto stärker ist die antimikrobielle Wirkung der Zusammensetzung gegen *Propionibakterium acnes.* Die Reduktionsfaktoren der Zubereitungen A bis C nach 24 h sind in Abbildung 1 dargestellt.

Der Reduktionsfaktor der Probe A wurde mit 3705 bestimmt. Somit weist die Probe A, die Octenidindihydrochlorid, Ethylhexylglycering und Propan-1,2-diol umfasst, nach 24h eine antimikrobielle Wirksamkeit gegen *Propionibakterium acnes* auf. Dagegen weist die Probe B, die ausschließlich Decan-1,2-diol enthält, im Vergleich zur Kontrolle kaum eine antimikrobielle Wirkung im Suspensionstest auf (Reduktionsfaktor 1). Überraschender Weise weist allerdings die Probe C, die Decan-1,2-diol, Octenidindihydrochlorid, Ethylhexylglycerin und Propan-1,2-diol umfasst, eine überaus effektive antimikrobielle Wirksamkeit gegen *Propionibakterium acnes* auf. Der bestimmte Reduktionsfaktor beträgt 113741. Da die Wirksamkeit der erfindungsgemäßen Probe C über die additive Wirksamkeit der Einzelsubstanzen hinausgeht, kann hier von einem Synergismus gesprochen werden.

Erfindungsgemäße dermatologische und/oder kosmetische Zubereitungen können in verschiedenen Formen vorliegen. So können diese Zubereitungen z. B. eine Lösung (z.B. wässrige, wässrig-alkoholische oder alkoholische Lösung), eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W) oder Öl-in-Wasser-in-Öl (O/W/O) (jeweils auch in Form von Silikonöl-Emulsionen), eine Hydrodispersion oder Lipodispersion, eine Pickering-Emulsion, einen festen Stift oder auch ein Aerosol darstellen. Auch ist die Verwendung in einer Zubereitung als Tränkung für ein wasserunlösliches Substrat (z.B. ein Tuch, ein Vlies, ein Pad) erfindungsgemäß vorteilhaft, wobei es sich bei dieser Applikationsform sowohl um ein trocken anmutendes als auch um ein feucht anmutendes Substrat handeln kann.

Erfindungsgemäß vorteilhafte Darreichungsformen der erfindungsgemäßen dermatologischen und/oder kosmetischen Zubereitung sind Cremes, Salben, Hydrodispersionen, Lotionen, Tinkturen, Pumpsprays, Aerosolsprays, wässrigen Lösungen, Reinigungssubstrate und dergleichen.

Vorteilhaft ist es ferner, die erfindungsgemäße dermatologische und/oder kosmetische Zubereitung als Reinigungsprodukt, Pflegeprodukt, Toner oder Peelingprodukt zu verwenden.

Es ist ferner vorteilhaft, wenn die erfindungsgemäße dermatologische und/oder kosmetische Zubereitung einen Wassergehalt von mehr als 30 Gew.-%, bevorzugt mehr als 60 Gew.-%, insbesondere bevorzugt mehr als 70 Gew.-% aufweist.

Die dermatologische und/oder kosmetische Zubereitung gemäß der Erfindung kann ferner kosmetische Hilfsstoffe und Wirkstoffe enthalten, wie sie üblicherweise in solchen Zubereitungen verwendet werden, z. B. Wirkstoffe, Konservierungsmittel, Konservierungshelfer, Bakterizide, Lipide, Substanzen zum Verhindern des Schäumens, Farbstoffe und Farbpigmente, Verdickungsmittel, anfeuchtende und/oder feuchthaltende Substanzen oder andere übliche Bestandteile einer kosmetischen oder dermatologischen Formulierung wie Polymere, Schaumstabilisatoren, organische Lösungsmittel, Lichtschutzfilter oder Silikonderivate, sofern der Zusatz die geforderten Eigenschaften hinsichtlich der Stabilität nicht beeinträchtigen oder ausgeschlossen sind.

Nachfolgende Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge

bzw. auf das Gesamtgewicht der Zubereitung bezogen.

**O/W Cremes:**

| INCI | 1 | 2 | 3 | 4 | 5 |
|---|---|---|---|---|---|
| Sodium Cetearyl Sulfate | | 0,20 | | | |
| Glyceryl Stearate Citrate | | | | | 2,00 |
| Glyceryl Stearate | 3,00 | | | 2,00 | |
| PEG-40 Stearate | 1,00 | | | 2,00 | |
| Sodium Stearoyl Glutamate | | | 0,30 | | |
| Glyceryl Stearate SE | | 2,00 | | | |
| PEG-150 Distearate | 0,30 | | 0,80 | | |
| Cetearyl Alcohol | | 2,00 | | | 2,00 |
| Stearyl Alcohol | 1,00 | | 1,00 | 1,00 | 1,00 |
| Cetyl Alcohol | 1,00 | | | | |
| Hydrogenated Coco Glycerides | | 1,00 | | | |
| Butyrospermum Parkii (Shea) Butter | 1,00 | 1,00 | 1,00 | | 5,00 |
| Xanthan Gum | 0,15 | | 0,20 | | |
| Acrylates/C10-30 Alkyl Acrylate Crosspolymer | 0,10 | 0,20 | | | |
| Cellulose | | | | 0,10 | |
| Ammonium Acryloyldimethyltaurate/VP Copolymer | 0,30 | | | 0,50 | 0,50 |
| Dimethicone | 5,00 | | 2,00 | | |
| Isopropyl Stearate | | 3,00 | 3,00 | | |
| C12-15 Alkyl Benzoate | | | | 4,00 | |
| Sodium Hyaluronate | | | 1,00 | | |
| Polyquaternium-10 | | | | | 1,00 |
| Hydrogenated Polydecene | | | | 1,00 | |
| Caprylic/Capric Triglyceride | | | 1,00 | | |
| Ethylhexylkokosfettsäureester | | 1,00 | | | |
| Füllstoffe / Additive (bspw. Stärke, Silica, BHT, Talc, Kaolin...) | 5,00 | | 7,00 | | 3,00 |
| Lichtfilter (bspw. Bis-ethylhexyloxyphenol Methoxyphenyl Triazine) | 5,00 | | | | 8,00 |
| Ubiquinone | | 0,10 | | | |
| Isobutylamido Thiazolyl Resorcinol | | | | 0,30 | |
| Butylen Glycol | | 2,00 | | | |
| Glycerin | 4,00 | 5,00 | 4,00 | 7,00 | 3,00 |
| Lactic Acid | | 3,00 | | | |
| Carnitine | 0,50 | | | | |
| Glycyrrhiza Inflata Root Extract | | 0,03 | | | |
| Parfum | | | | 0,30 | |
| Trisodium EDTA | | 1,00 | | | 1,00 |

(fortgesetzt)

| INCI | 1 | 2 | 3 | 4 | 5 |
|---|---|---|---|---|---|
| Ethanol | 4,00 | | | | |
| Benzophenone-4 | | 0,10 | | | |
| Phenoxyethanol | 0,20 | | 0,60 | 0,40 | |
| Caprylyl Glycol (Octan-1,2-diol) | 0,05 | | | | |
| 1,2-Decandiol (Decan-1,2-diol) | | 0,10 | | | |
| Lauryl Glycol (Dodecan-1,2-diol) | | | 0,50 | 0,30 | 0,50 |
| 20 Gew.-% Octenidindihydrochlorid + 10 Gew.-% Ethylhexylglycerin + 70 Gew.-% Propan-1,2-diol | 0,10 | 0,30 | 0,05 | 0,10 | 0,50 |
| Aqua | Ad 100 | | | | |

**O/W Cremes:**

| INCI | 6 | 7 | 8 | 9 | 10 | 11 |
|---|---|---|---|---|---|---|
| Steareth-21 | | | 2,00 | | | |
| Steareth-2 | | | 1,00 | | | |
| Sorbitan Stearate | | 1,00 | | | | |
| Polyglycerol Methyl Glucose Distearate | | 3,00 | | | | |
| PEG-40 Stearate | 1,00 | | | | | |
| Glyceryl Stearate SE | 3,00 | | | | | |
| PEG-100 Stearate | | | | | 2,00 | |
| PEG-150 Distearate | | | | | 2,00 | |
| Cetearyl Glucoside | | | | 3,00 | | |
| Stearic Acid | | | | | | 2,50 |
| Cetearyl Alcohol | 2,00 | | | | | 2,00 |
| Stearyl Alcohol | | | | | | 1,00 |
| Myristyl Myristate | | | 1,50 | | | |
| Cetyl Alcohol | | 2,50 | | | | |
| Hydrogenated Coco Glycerides | | | 1,00 | | | |
| Butyrospermum Parkii (Shea) Butter | | 3,00 | | 1,00 | | |
| Carbomer (Polyacrylsäure) | | | | | 0,50 | 0,40 |
| Xanthan Gum | | 0,15 | | | | |
| Acrylates/C10-30 Alkyl Acrylate Crosspolymer | 0,10 | | 0,30 | | | |
| Cellulose | | | | 2,00 | | |
| Ammonium Acryloyldimethyltaurate/VP Copolymer | | | | 0,20 | | |
| Isopropyl Stearate | | 2,00 | | | | |
| Octyldodecanol | 5,00 | | 1,00 | | | |
| C12-15 Alkyl Benzoate | | 1,00 | 5,00 | 1,00 | | |

(fortgesetzt)

| INCI | 6 | 7 | 8 | 9 | 10 | 11 |
|---|---|---|---|---|---|---|
| PEG-40 Hydrogenated Castor Oil | | | | | | 0,50 |
| Hydrogenated Polydecene | | | 2,00 | | | |
| Dicaprylyl Ether | 1,00 | | | 2,00 | | |
| Füllstoffe / Additive (bspw. Stärke, Silica, BHT, Talc, Kaolin...) | | | | 10,0 | | |
| Pigmente (Bspw. Titanium Dioxide, CI 77491, CI 77492, CI 77499) | 5,00 | | 10,0 | | | |
| Lichtfilter (bspw. Bis-ethylhexyloxyphenol Methoxyphenyl Triazine) | | | 30,0 | | 20,0 | |
| Butylenglycol | | | 4,00 | | | |
| Glycerin | 3,00 | 5,00 | 4,00 | 3,00 | 3,00 | 5,00 |
| Lactic Acid | 2,00 | | | 5,00 | | |
| Carnitine | | 2,00 | | | | |
| Glycyrrhiza Inflata Root Extract | | | | | | 0,10 |
| Parfum | | | 0,20 | | | 0,10 |
| Trisodium EDTA | | | 0,50 | | | |
| Ethanol | | 3,00 | | | | |
| Phenoxyethanol | 0,60 | | 0,70 | | 0,80 | |
| Caprylyl Glycol (Octan-1,2-diol) | 0,05 | | | 0,5 | | |
| 1,2-Decandiol (Decan-1,2-diol) | | 0,05 | | | 0,5 | |
| Lauryl Glycol (Dodecan-1,2-diol) | | | 0,10 | | | 0,30 |
| 20 Gew.-% Octenidindihydrochlorid + 10 Gew.-% Ethylhexylglycerin + 70 Gew.-% Propan-1,2-diol | 0,50 | 0,05 | 0,30 | 0,01 | 0,10 | 0,20 |
| 2-Methyl-1,3 -Propandiol | | | | | | 0,20 |
| Aqua | ad 100 | | | | | |

**Reinigungszubereitungen:**

| INCI | 12 | 13 |
|---|---|---|
| PEG-40 Hydrogenated Castor Oil | 0,50 | |
| Decyl Glucoside | 0,10 | |
| Arginine HCL | 0,40 | 0,20 |
| Sodium Hyaluronate | | 1,00 |
| Poloxamer 124 | 2,00 | 3,00 |
| Pigmente (Bspw. Titanium Dioxide, CI 77491, CI 77492, CI 77499) | | 0,10 |
| Wasser- und/oder öllösliche Farbstoffe | 0,10 | |
| Butylene Glycol | 4,00 | |
| Glycerin | 2,00 | 5,00 |
| Sodium Hydroxide ad pH | | 0,20 |
| Lactic Acid | 1,50 | |

(fortgesetzt)

| INCI | 12 | 13 |
|---|---|---|
| Trisodium EDTA | | 0,50 |
| Ethanol | 15,0 | |
| Phenoxyethanol | 0,30 | 0,80 |
| Caprylyl Glycol (Octan-1,2-diol) | 0,20 | |
| 1,2-Decandiol (Decan-1,2-diol) | | 0,50 |
| Lauryl Glycol (Dodecan-1,2-diol) | 0,01 | |
| 20 Gew.-% Octenidindihydrochlorid + 10 Gew.-% Ethylhexylglycerin + 70 Gew.-% Propan-1,2-diol | 0,01 | 0,50 |
| 2-Methyl-1,3 -Propandiol | 0,30 | 0,10 |
| Aqua | ad 100 | |

**Reinigungszubereitungen:**

| INCI | 14 | 15 |
|---|---|---|
| Cocoamidopropyl Betaine | 3,00 | 1,00 |
| Coco-Glucoside | 4,00 | 2,00 |
| Xanthan Gum | 3,00 | |
| Acrylates/C10-30 Alkyl Acrylate Crosspolymer | 1,00 | |
| Dimethicone | 1,00 | 4,00 |
| Isopropyl Stearate | 2,00 | 1,00 |
| C12-15 Alkyl Benzoate | 2,00 | 2,00 |
| Pigmente (Bspw. Titanium Dioxide, CI 77491, CI 77492, CI 77499) | | 0,50 |
| Wasser- und/oder öllösliche Farbstoffe | 0,20 | |
| Glycerin | 3,00 | 3,00 |
| Benzophenone-4 | 0,10 | 0,10 |
| Caprylyl Glycol (Octan-1,2-diol) | 0,01 | |
| 1,2-Decandiol (Decan-1,2-diol) | | 0,10 |
| Lauryl Glycol (Dodecan-1,2-diol) | 0,50 | |
| 20 Gew.-% Octenidindihydrochlorid + 10 Gew.-% Ethylhexylglycerin + 70 Gew.-% Propan-1,2-diol | 0,50 | 0,01 |
| 2-Methyl-1,3 -Propandiol | 0,05 | 0,05 |
| Aqua | ad 100 | |

**Peelings:**

| INCI | 16 |
|---|---|
| Cocoamidopropyl betain | 10,0 |
| Coco-Glucoside | 5,00 |

(fortgesetzt)

| INCI | 16 |
|---|---|
| Acrylates/C10-30 Alkyl Acrylate Crosspolymer | 2,00 |
| Polyquaternium-10 | 0,20 |
| Wasser- und/oder öllösliche Farbstoffe | 0,10 |
| Glycerin | 1,00 |
| Sodium Hydroxide ad pH | 0,10 |
| Lactic Acid | 2,00 |
| Sodium Benzoate | 0,60 |
| Phenoxyethanol | 0,50 |
| Caprylyl Glycol (Octan-1,2-diol) | 0,10 |
| 1,2-Decandiol (Decan-1,2-diol) | 0,30 |
| Lauryl Glycol (Dodecan-1,2-diol) | 0,05 |
| 20 Gew.-% Octenidindihydrochlorid + 10 Gew.-% Ethylhexylglycerin + 70 Gew.-% Propan-1,2-diol | 0,10 |
| 2-Methyl-1,3 -Propandiol | 0,10 |
| Aqua | ad 100 |

**Patentansprüche**

1. Dermatologische und/oder kosmetische Zubereitung, umfassend

   a) ein oder mehrere Bispyridiniumalkane,
   b) ein oder mehrere 1- oder 2-($C_1$ bis $C_{24}$-Alkyl)-glycerinether,
   c) ein oder mehrere Polyole mit einer Kettenlänge von maximal 4 Kohlenstoffatomen, und
   d) ein oder mehrere Alkan-1,2-diolen mit einer Kettenlänge von 8 bis 12 Kohlenstoffatomen,

   **dadurch gekennzeichnet ist, dass** die dermatologische und/oder kosmetische Zubereitung frei von aluminiumhaltigen antitranspirant wirkenden Substanzen ist und dass das Gewichtsverhältnis der Bispyridiniumalkane zu den 1- oder 2-($C_1$ bis $C_{24}$-Alkyl)-glycerinethern von 3:1 bis 1,7:1 beträgt.

2. Dermatologische und/oder kosmetische Zubereitung nach Anspruch 1 **dadurch gekennzeichnet, dass** diese frei von Triclosan ist.

3. Dermatologische und/oder kosmetische Zubereitung nach mindestens einem der vorstehenden Ansprüche **dadurch gekennzeichnet, dass** Bispyridiniumalkane in einem Gesamtanteil von 0,001 Gew.-% bis 1,0 Gew.-%, bevorzugt von 0,01 Gew.-% bis 0,5 Gew.-%, insbesondere bevorzugt 0,05 Gew.-% bis 0,2 Gew.-%, bezogen auf das Gesamtgewicht der Zubereitung, enthalten sind.

4. Dermatologische und/oder kosmetische Zubereitung nach mindestens einem der vorstehenden Ansprüche **dadurch gekennzeichnet, dass** als Bispyridiniumalkan Octenidindihydrochlorid gewählt wird.

5. Dermatologische und/oder kosmetische Zubereitung nach mindestens einem der vorstehenden Ansprüche **dadurch gekennzeichnet, dass** der Gesamtanteil der 1- oder 2-($C_1$ bis $C_{24}$-Alkyl)-glycerinether 0,001 Gew.-% bis 1,0 Gew.-%, bevorzugt 0,01 Gew.-% bis 0,5 Gew.-%, insbesondere bevorzugt 0,02 Gew.-% bis 0,1 Gew.-%, bezogen auf das Gesamtgewicht der Zubereitung, beträgt.

6. Dermatologische und/oder kosmetische Zubereitung nach mindestens einem der vorstehenden Ansprüche **dadurch gekennzeichnet, dass** als 1- oder 2-($C_1$ bis $C_{24}$-Alkyl)-glycerinether Ethylhexylglycerin gewählt wird.

**7.** Dermatologische und/oder kosmetische Zubereitung nach mindestens einem der vorstehenden Ansprüche **dadurch gekennzeichnet, dass** als Polyole mit einer Kettenlänge von maximal 4 Kohlenstoffatomen Glycerin und/oder Propan-1,2-diol gewählt werden.

**8.** Dermatologische und/oder kosmetische Zubereitung nach mindestens einem der vorstehenden Ansprüche **dadurch gekennzeichnet, dass** Alkan-1,2-diole mit einer Kettenlänge von 8 bis 12 Kohlenstoffatomen in einem Gesamtanteil von 0,01 Gew.-% bis 1 Gew.-%, bevorzugt von 0,05 Gew.-% bis 0,7 Gew.-% und insbesondere besonders von 0,1 Gew.-% bis 0,5 Gew.-%, bezogen auf das Gesamtgewicht der Zubereitung, enthalten sind.,

**9.** Dermatologische und/oder kosmetische Zubereitung nach mindestens einem der vorstehenden Ansprüche **dadurch gekennzeichnet, dass** als ein oder mehrere Alkan-1,2-diole mit einer Kettenlänge von 8 bis 12 Kohlenstoffatomen Octan-1,2-diol, Decan-1,2-diol und/oder Dodecan-1,2-diol gewählt werden.

## Claims

**1.** Dermatological and/or cosmetic preparation comprising

a) one or more bispyridinium alkanes,
b) one or more 1- or 2-($C_1$ to $C_{24}$-alkyl) glycerin ethers,
c) one or more polyols having a chain length of at most 4 carbon atoms, and
d) one or more alkane-1,2-diols having a chain length of 8 to 12 carbon atoms,

**characterized in that** the dermatological and/or cosmetic preparation is free from aluminium-containing antiperspirant substances and **in that** the ratio by weight of the bispyridinium alkanes to the 1- or 2-($C_1$ to $C_{24}$-alkyl) glycerin ethers is from 3:1 to 1.7:1.

**2.** Dermatological and/or cosmetic preparation according to Claim 1, **characterized in that** said preparation is free from triclosan.

**3.** Dermatological and/or cosmetic preparation according to at least one of the preceding claims, **characterized in that** bispyridinium alkanes are present at an overall proportion of 0.001% by weight to 1.0% by weight, preferably of 0.01% by weight to 0.5% by weight, particularly preferably 0.05% by weight to 0.2% by weight, based on the total weight of the preparation.

**4.** Dermatological and/or cosmetic preparation according to at least one of the preceding claims, **characterized in that** octenidine dihydrochloride is selected as the bispyridinium alkane.

**5.** Dermatological and/or cosmetic preparation according to at least one of the preceding claims, **characterized in that** the overall proportion of the 1- or 2-($C_1$ to $C_{24}$-alkyl) glycerin ethers is 0.001% by weight to 1.0% .by weight, preferably 0.01% by weight to 0.5% by weight, particularly preferably 0.02% by weight to 0.1% by weight, based on the total weight of the preparation.

**6.** Dermatological and/or cosmetic preparation according to at least one of the preceding claims, **characterized in that** ethylhexylglycerin is selected as the 1- or 2-($C_1$ to $C_{24}$-alkyl) glycerin ether.

**7.** Dermatological and/or cosmetic preparation according to at least one of the preceding claims, **characterized in that** glycerin and/or propane-1,2-diol are selected as the polyols having a chain length of at most 4 carbon atoms.

**8.** Dermatological and/or cosmetic preparation according to at least one of the preceding claims, **characterized in that** alkane-1,2-diols having a chain length of 8 to 12 carbon atoms are present at an overall proportion of 0.01% by weight to 1% by weight, preferably of 0.05% by weight to 0.7% by weight and particularly preferably of 0.1% by weight to 0.5% by weight, based on the total weight of the preparation.

**9.** Dermatological and/or cosmetic preparation according to at least one of the preceding claims, **characterized in that** octane-1,2-diol, decane-1,2-diol and/or dodecane-1,2-diol are selected as the one or more alkane-1,2-diols having a chain length of 8 to 12 carbon atoms.

## Revendications

1. Préparation dermatologique et/ou cosmétique, comprenant

   a) un ou plusieurs bispyridiniumalcanes,
   b) un ou plusieurs éthers 1- ou 2-(alkyliques en $C_1$ à $C_{24}$) du glycérol,
   c) un ou plusieurs polyols ayant une longueur de chaîne au maximum de 4 atomes de carbone, et
   d) un ou plusieurs alcane-1,2-diols ayant une longueur de chaîne de 8 à 12 atomes de carbone,

   **caractérisée en ce que** la préparation dermatologique et/ou cosmétique est exempte de substance à effet antitranspirant contenant de l'aluminium et **en ce que** le rapport en poids des bispyridiniumalcanes aux éthers 1- ou 2-(alkyliques en $C_1$ à $C_{24}$) du glycérol est de 3:1 à 1,7:1.

2. Préparation dermatologique et/ou cosmétique selon la revendication 1, **caractérisée en ce qu'**elle est exempte de triclosane.

3. Préparation dermatologique et/ou cosmétique selon au moins l'une des revendications précédentes, **caractérisée en ce qu'**elle contient des bispyridiniumalcanes selon une proportion totale de 0,001 % en poids à 1,0 % en poids, de préférence de 0,01 % en poids à 0,5 % en poids, de manière particulièrement préférée de 0,05 % en poids à 0,2 % en poids, par rapport au poids total de la composition.

4. Préparation dermatologique et/ou cosmétique selon au moins l'une des revendications précédentes, **caractérisée en ce qu'**on choisit comme bispyridiniumalcane le dichlorhydrate d'octénidine.

5. Préparation dermatologique et/ou cosmétique selon au moins l'une des revendications précédentes, **caractérisée en ce que** la proportion totale des éthers 1- ou 2- (alkyliques en $C_1$ à $C_{24}$) du glycérol est de 0,001 % en poids à 1,0 % en poids, de préférence de 0,01 % en poids à 0,5 % en poids, de manière particulièrement préférée de 0,02 % en poids à 0,1 % eh poids, par rapport au poids total de la préparation.

6. Préparation dermatologique et/ou cosmétique selon au moins l'une des revendications précédentes, **caractérisée en ce qu'**on choisit comme éther 1- ou 2-(alkyliques en $C_1$ à $C_{24}$) du glycérol l'éthylhexylglycérol.

7. Préparation dermatologique et/ou cosmétique selon au moins l'une des revendications précédentes, **caractérisée en ce qu'**on choisit comme polyols ayant une longueur de chaîne au maximum de 4 atomes de carbone le glycérol et/ou le propane-1,2-diol.

8. Préparation dermatologique et/ou cosmétique selon au moins l'une des revendications précédentes, **caractérisée en ce qu'**elle contient des alcane-1,2-diols ayant une longueur de chaîne de 8 à 12 atomes de carbone' selon une proportion totale de 0,01 % en poids à 1 % en poids, de préférence de 0,05 % en poids à 0,7 % en poids et de manière particulièrement préférée de 0,1 % en poids à 0,5 % en poids, par rapport au poids total de la préparation.

9. Préparation dermatologique et/ou cosmétique selon au moins l'une des revendications précédentes, **caractérisée en ce qu'**on choisit comme un ou plusieurs alcanes-1,2-diols ayant une longueur de chaîne de 8 à 12 atomes de carbone l'octane-1,2-diol, le décane-1,2-diol et/ou le dodécane-1,2-diol.

Abbildung 1:

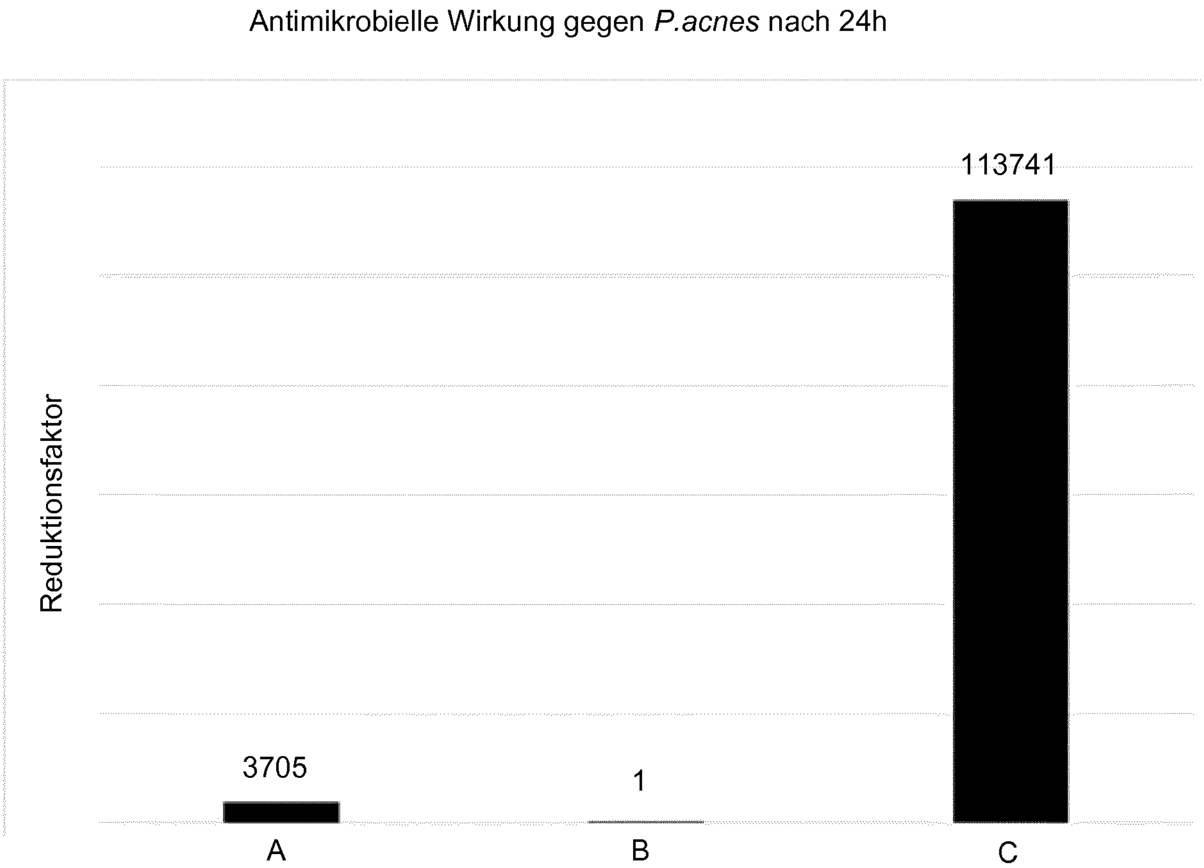
Antimikrobielle Wirkung gegen *P.acnes* nach 24h
Reduktionsfaktor
3705
1
113741
A
B
C

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102005002644 A1
- JP 20022003330 B
- WO 03000220 A2
- US 6123953 A
- EP 1915982 A1
- EP 1598064 A1
- WO 2013167220 A1
- DE 102008011692 A1


### In der Beschreibung aufgeführte Nicht-Patentliteratur


- Kosmetik, Entwicklung, Herstellung und Anwendung kosmetischer Mittel. Thieme Verlag, 1995
- **PSCHYREMBEL.** Klinisches Wörterbuch. Walter de Gruyter-Verlag, 1998